# EUROPÄISCHE PATENTSCHRIFT

(11) **EP 2 640 289 B1**
(45) Veröffentlichungstag und Bekanntmachung des Hinweises auf die Patenterteilung: **14.09.2016**
(21) Anmeldenummer: 11785601.3
(22) Anmeldetag: 18.11.2011
(51) Int. Cl.: A61F 2/46, A61F 2/82, A61B 17/70, A61B 17/16, A61B 17/34, A61B 17/86, A61B 17/00, A61F 2/30, A61F 2/44, A61M 25/01, A61M 25/10

(54) **VORRICHTUNG ZUM VERBINDEN KNÖCHENER STRUKTUREN**
DEVICE FOR CONNECTING BONY STRUCTURES
DISPOSITIF POUR LA LIAISON DE STRUCTURES OSSEUSES

(30) Priorität: 20.11.2010 DE 102010052113
(43) Veröffentlichungstag der Anmeldung: 25.09.2013
(73) Patentinhaber: Strecker, Ernst-Peter, 76228 Karlsruhe (DE)
(72) Erfinder: Strecker, Ernst-Peter, 76228 Karlsruhe (DE)
(74) Vertreter: von Hellfeld, Axel
(86) Internationale Anmeldenummer: PCT/EP2011/005828
(87) Internationale Veröffentlichungsnummer: WO 2012/065753

(56) Entgegenhaltungen:
- WO-A1-00/33909
- WO-A2-2006/088776
- US-A- 6 127 597
- US-A1- 2004 097 927
- US-A1- 2007 083 205
- US-A1- 2009 062 868
- US-A1- 2010 016 903

## Beschreibung

Die Erfindung betrifft eine Vorrichtung zum Verbinden von Knochen, insbesondere von menschlichen Wirbelkörpern.

### Zum Stand der Technik

Rückenschmerzen können sowohl die Lebensqualität als auch die Arbeitsleistung von Menschen stark beeinträchtigen, insbesondere bei Menschen, die berufsmäßig über längere Zeit stehen oder schwere Lasten heben müssen. Hervorgerufen werden Rückenschmerzen sehr häufig durch Bandscheibenschädigungen, jedoch auch durch Verschiebung von Wirbelkörpern gegeneinander, dem sogenannten Wirbelgleiten, durch Instabilität der Wirbelsäule mit vermehrter Beweglichkeit zwischen einzelnen Wirbelkörpern, oder einen sogenannten engen Wirbelkanal, der durch knöcherne Anbauten und Bandscheibenvorwölbungen und Bandscheibenvorfälle bedingt sein kann.

Bei einer Vielzahl von Patienten ist deshalb eine operative Verbindung der beiden das erkrankte Segment betreffenden Wirbelkörper angezeigt. Im Stand der Technik bedeutet eine solche sogenannte Wirbelfusion jedoch immer eine sehr große Operation. Dabei ist in der Regel sowohl ein anteriorer und ein posteriorer Zugang notwendig, d.h. es muss offen über die Bauchhöhle und über den Rücken operiert werden. Solche Operationen erstrecken sich häufig über Stunden und sind allein schon deshalb vielen Patienten nicht zumutbar. Auch können bei solch umfassenden Eingriffen schwere Komplikationen auftreten. Nach einer solchen Behandlung ist zumeist eine lange Rehabilitationsphase erforderlich und trotzdem bleiben viele Patienten arbeitsunfähig.

Die US 2004/0092933 A1 zeigt einen biegsamen Bohrer, der in zwei benachbarte Wirbelkörper einen Kanal bohrt. Es wird damit eine Kavität in der Bandscheibe erzeugt und in der Kavität wird eine Spreizeinrichtung positioniert. Eine Wirbelfusion findet dort allenfalls mit einer Art Zement- oder Knochenimplantat statt.

Die WO 00/33909 zeigt eine gebogene Hohlnadel, die adaptiert ist, um in den Knochenmarkbereich eines Wirbels eingeschoben zu werden. Die Nadel ist nicht adaptiert, um in härtere Bereiche eines Knochens, z. B. die Kortikalis, insbesondere eines Wirbels, eingeschlagen zu werden.

Eine medizinische Vorrichtung mit den Merkmalen des Oberbegriffs des Anspruchs 1 ist aus der WO 2006/088776 A2 bekannt.

### Kurzbeschreibung der Erfindung

Die vorliegende Erfindung hat zum Ziel, eine perkutane, minimal-invasive Möglichkeit zu eröffnen, um Knochen und Knochenzwischenräume zu behandeln und um insbesondere Knochen, wie zum Beispiel Wirbelkörper, zu fusionieren und starr zu verbinden.

Die erfindungsgemäße Lösung liegt in den Merkmalen des unabhängigen Anspruchs 1. Vorteilhafte Weiterbildungen der Erfindung sind Gegenstand der abhängigen Ansprüche.

Hierzu stellt die Erfindung einen Nagel bereit, der ausgebildet ist zum perkutanen Einbringen in zumindest einen Knochen, wobei der Nagel eingerichtet ist, eine erste Gestalt anzunehmen, in der er gerade in einem Einführrohr positionierbar ist, und eine zweite Gestalt, in der er außerhalb des Einführrohres eine gekrümmte Form annimmt, und wobei der Nagel an seinem distalen Ende.oder über seine gesamte Länge ein Gewinde hat

Mit einem solchen Nagel ist es insbesondere möglich, bei krankhaft vermehrter Beweglichkeit von Wirbelkörpern, minimal-invasiv eine perkutane Wirbelkörperfusion durchzuführen.

Hierzu ist der genannte Nagel hinsichtlich seiner Abmessungen, seines Materials und seiner Gestalt so gewählt, dass er über eine Wirbelkörperseitenwand in Richtung angenähert senkrecht zur lokalen Wirbelsäulenachse in einen ersten Wirbelkörper einschlagbar ist (zum Beispiel mit einem Hammer), wobei der Nagel dann beim Einschlagen eine gekrümmte Form annimmt und den genannten ersten Wirbelkörper durchsetzt, die Bandscheibe durchdringt und sodann über die Deckplatte des benachbarten Wirbelkörpers in diesen eindringt, um eine feste Verbindung zwischen diesen beiden Knochen (Wirbelkörpern) herzustellen.

Gemäß einer bevorzugten Ausgestaltung ist der Nagel aufgrund einer internen Eigenspannung von der ersten Gestalt in die zweite Gestalt bringbar. Die Eigenspannung kann eine elastische oder auch superelastische Biegespannung sein. Die Eigenspannung kann auch temperaturabhängig sein, insbesondere bei Verwendung eines Metalls mit sogenanntem Formgedächtnis.

Dient der Nagel der vorstehend skizzierten Fusion von Knochen, insbesondere einer Wirbelkörperfusion, ist er massiv, jedoch nicht erfindungsgemäß, d.h. ohne Lumen oder dergleichen.

Wird andererseits der Nagel eingesetzt, um einen Knochen, zum Beispiel einen Wirbelkörper, im Inneren zu behandeln oder etwas in das Innere des Knochens einzubringen oder dient der Nagel dazu, einen Zwischenraum zwischen Knochen, wie zum Beispiel den Raum zwischen Wirbelkörpern, zu behandeln oder etwas in diesen Raum einzubringen, dann ist der Nagel gemäß der vorliegenden Erfindung hohl.

Je nach Einsatzzweck hat der Nagel einen Durchmesser von zum Beispiel 1 bis 8 mm. Er besteht bevorzugt aus einem Metall oder einer Metalllegierung, insbesondere mit superelastischen Eigenschaften.

Eine weitere nicht erfindungsgemäße Ausgestaltung des Nagels sieht vor, dass er an seinem distalen Ende angespitzt ist, und zwar in Form eines schrägen Zylinderschnittes derart, dass die distale Spitze in Bezug auf eine bogenförmige Krümmung des Nagels in seiner zweiten Gestalt auf der konvexen Seite der Krümmung liegt. Damit wird erreicht, dass beim oben erläuterten Einschlagen des Nagels in den Knochen der Nagel nach Verlassen des Einführrohres nicht nur aufgrund seiner Eigenspannung in die zweite Gestalt übergeht, sondern zusätzlich aufgrund der Wechselwirkung zwischen seiner Spitze und dem Knochenmaterial eine Ablenkung erfährt, welche die genannte Krümmung in der zweiten Gestalt noch verstärkt (im Sinne einer Reduzierung des Krümmungsradius). Diese beim Einschlagen des Nagels in das Knochenmaterial entstehende Zwangskrümmung erzeugt eine Verspannung des Nagels in dem von ihm selbst erzeugten Knochenkanal und dadurch wird der stabile Sitz des Nagels im Knochen sowie gegebenenfalls die Stabilität der Knochenfusion, insbesondere der Wirbelkörperfusion, gefördert.

Beschrieben wird auch das Einbringen eines Implantates oder von Substanzen in einen Knochen oder in Räume zwischen Knochen, wie Räume zwischen zwei benachbarten Wirbeln. Bei einem solchen Implantat kann es sich zum Beispiel um einen expandierbaren (zum Beispiel mittels Ballon expandierbaren) oder selbstexpandierenden Stütz- oder Spreizkörper handeln oder auch um aushärtende Substanzen, genetisch wirkende Substanzen, Medikamente, Zellen oder Zellverbände.

Auch kann die Bandscheibe insgesamt oder teilweise entfernt werden, z. B. durch chemische Auflösung, Mazeration, Lasereinwirkung, Zangenextraktion oder Aspiration.

Weitere Merkmale und Vorteile der Erfindung ergeben sich aus der nachfolgenden Beschreibung von Ausführungsbeispielen. Es zeigen:
- Figur 1: schematisch einen Schnitt durch den Wirbelpedikel und Wirbelkörper eines ersten Ausführungsbeispiels eines in einen Wirbelkörper eingebrachten Einführrohres mit einem Nagel und einem Pusher (Schieber);
- Figur 2: die Anordnung gemäß Fig. 1 in einem Stadium, in dem der Nagel in zwei zu fusionierende Wirbelkörper eingetrieben ist;
- Figur 3: eine axiale Draufsicht auf Einführrohr und Nagel gemäß Fig. 1 in Bezug auf den Wirbel in Draufsicht (axial von oben);
- Figur 4: ein Beispiel, bei dem der Nagel als Hohlnagel ausgebildet ist, in den ein Schlauch eingeführt ist;
- Figur 5: eine Kopplung zwischen Nagel und Pusher derart, dass ein Benutzer aufgrund der Stellung des Pushers den Drehwinkel des Nagels und damit die Richtung der Krümmung erkennt;
- Figur 6: ein Beispiel mit hohlem Nagel, durch den ein selbstexpandierender Abstandhalter (Spacer) in einen Zwischenraum zwischen benachbarten Wirbeln eingeschoben wird;
- Figur 7: einen weiter fortgeschrittenen Zustand des Systems gemäß Fig. 6, wobei der Abstandshalter in den Zwischenwirbelraum eingeführt ist;
- Figur 8: ein Einführrohr mit Nagel und Abstandshalter, wobei der Nagel durch den Abstandshalter gemäß Fig. 7 geschoben ist und zwei benachbarte Wirbelkörper miteinander verbindet;
- Figur 9: eine Anordnung ähnlich Fig. 7, wobei nicht ein selbstexpandierender Abstandshalter sondern ein ballon-expandierbarer Abstandshalter eingesetzt ist;
- Figur 10: eine Variante des Beispieles gemäß Fig. 9, wobei der Abstandshalter durch Endplatten der benachbarten Wirbelkörper greift;
- Figur 11: verschiedene Varianten von Querschnitten des Nagels;
- Figur 12: einen Nagel mit einem distalen Kopfende, dessen Durchmesser im Vergleich zum übrigen Nagel vergrößert ist;
- Figur 13: einen Nagel mit Ankern;
- Figur 14: ein Einführrohr mit einem Nagel, wobei das Einführrohr an seinem distalen Ende eine zweite Gestalt annehmen kann, der Nagel ist zum Einschlagen starr;
- Figur 15: ein Beispiel ähnlich Fig. 14, wobei der Nagel dahingehend abgewandelt ist, dass er eine zweite Gestalt annehmen kann;
- Figur 16: ein Einführrohr mit Nagel, wobei sowohl das Einführrohr als auch der Nagel die erste Gestalt und die zweite Gestalt annehmen;
- Figur 17: eine Ausgestaltung eines Nagels mit einem Bereich reduzierten Durchmessers nahe dessen distalem Ende;
- Figur 18: eine Variante des Nagels gemäß Fig. 17 mit einer Kunststoffhülle in dem Bereich mit reduziertem Durchmesser;
- Figur 19: den Zustand des Systems gemäß zum Beispiel Fig. 18 nach Entfernen des zentralen Nagels;
- Figur 20: eine Draufsicht auf einen Wirbel, dessen Wirbelkörper mittels Abstandhaltern (Spacern) stabilisiert ist;
- Figur 21: eine Draufsicht auf einen Wirbel, dessen Wirbelkörper mit zwei konzentrisch angeordneten Abstandshaltern abgestützt ist;
- Figur 22: den Einsatz eines Nagels bei einer Wirbelkörperfraktur;
- Figur 23: den Einsatz von zwei Nägeln mit einem "Fixateur Interne";
- Figur 24: ein erfindungsgemäße Ausführungsbeispiel, bei dem ein äußerer Nagel und ein innerer Nagel vorgesehen sind, wobei der äußere Nagel an seinem distalen Ende mit einem Gewinde versehen ist, und
- Figur 25: eine Detailansicht eines äußeren Nagels gemäß Fig. 24.

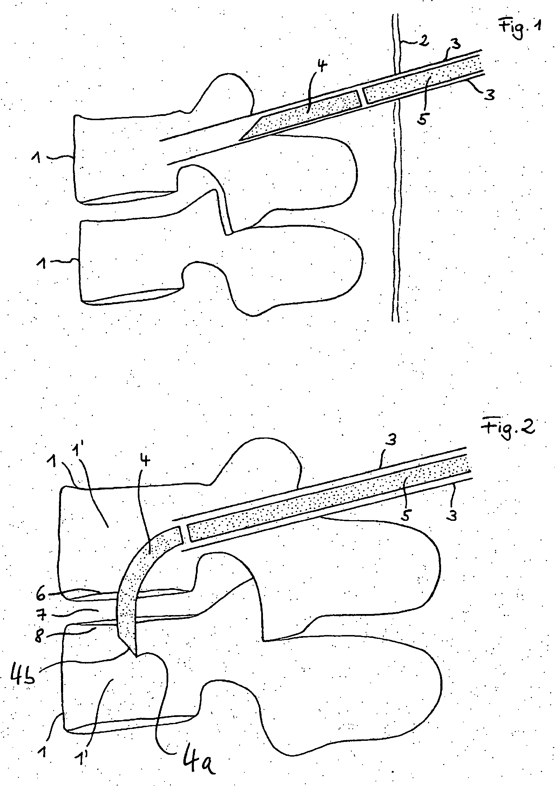

Die Figuren 1, 2 und 3 zeigen ein erstes Beispiel einer Vorrichtung zum Verbinden knöcherner Strukturen, hier zum Verbinden zweier benachbarter Wirbelkörper 1. Die Figuren 1 und 2 zeigen schematische eine Ansicht lateral von der Seite, also senkrecht zur lokalen Wirbelsäulenachse. In den Figuren 1 und 2 ist dorsal rechts und der Eingriff erfolgt perkutan, also durch die Haut 2.

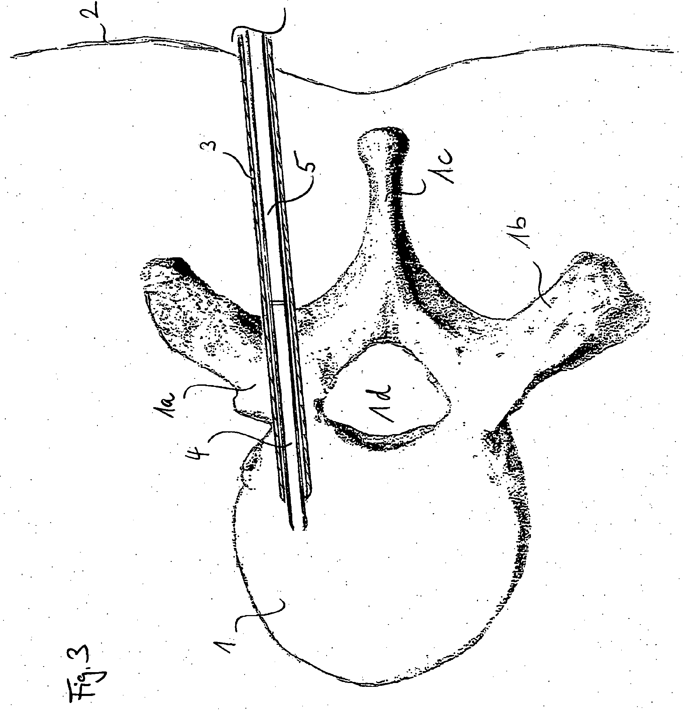

Hierzu wird ein langgestrecktes und gerades Einführrohr 3 aus z.B. Metall transpedikulär, also durch das Pedikel 1a (siehe Fig. 3) des Wirbelbogens bis in den Wirbelkörper 1 vorgeschoben. Fig. 3 zeigt noch in Draufsicht den Querfortsatz 1b, den Dornfortsatz 1c und den Wirbelkanal 1d. Auch der Querfortsatz 1b kann als Zugang zum Wirbelkörper über ein eingebrachtes Einführrohr 3 genutzt werden.

Gemäß den Figuren 1, 2 und 3 gleitet in dem Einführrohr 3 ein Nagel 4, der mittels eines Pushers (Schiebers) 5 vorschiebbar ist und so gemäß den Figuren 1 und 2 aus dem offenen distalen Ende des geraden Einführrohres 3 heraustreibbar ist. Das Einführrohr 3 ist an seinem distalen Ende geschärft. Es kann gegebenenfalls wie ein Tubus mit einem Mandrin (nicht gezeigt) eingetrieben werden. Das Einführrohr ähnelt einer Kanüle, wie sie in der Vertebroplastie eingesetzt wird. Dieses Eintreiben und auch das Eintreiben bei allen nachfolgend beschriebenen Vorgängen erfolgt unter Röntgendurchleuchtungskontrolle oder unter Röntgencomputertomographie. Das Einführrohr kann, je nach Diagnose und Indikation, einen Außendurchmesser im Bereich von etwa 1 bis etwa 8 mm aufweisen. Es ist vorne spitz angeschliffen, insbesondere kann die Spitze in Form eines schrägen Zylinderschnittes gestaltet sein.

Der Nagel 4 beim Beispiel gemäß den Figuren 1 bis 3 ist massiv, d.h. ohne Lumen oder dergleichen. Der Nagel 4 dient hier einer Fusion der Wirbelkörper. Hierzu wird der Nagel 4 gemäß Fig. 2 mit dem Pusher 5 aus dem Einführrohr getrieben (mit zum Beispiel einem Hammer, nicht gezeigt). Der Nagel 4 hat gemäß Fig. 1 im geraden Einführrohr eine erste Gestalt, die aufgrund der Zwangskräfte des Einführrohres ebenfalls gerade langgestreckt ist. Wirken keine äußeren Zwangskräfte auf den Nagel 4 ein, nimmt er von selbst eine zweite Gestalt in gekrümmter Bogenform an. Der Übergang von der ersten in die zweite Gestalt kann zum Beispiel dadurch erfolgen, dass der metallische Nagel 4 ohne äußere Zwangskräfte bogenförmig gekrümmt ist und nur im Einführrohr aufgrund der passgenauen Führung im Rohr zwangsweise langgestreckt geradlinig ist. Der Übergang von der ersten in die zweite Gestalt kann aber auch durch thermische Effekte erreicht werden, zum Beispiel bei Verwendung einer Metalllegierung mit Formgedächtnis, zum Beispiel Nitinol, insbesondere einer Legierung mit superelastischen Eigenschaften.

Der sich beim Austritt aus dem Einführrohr 3 gemäß Fig. 2 krümmende Nagel wird durch die Grundplatte 6 des in den Figuren oben dargestellten Wirbelkörpers 1 getrieben, sodann durch den Zwischenwirbelraum 7 (Bandscheibenfach), weiterhin dann durch die Deckplatte 8 des in den Figuren unten dargestellten Wirbelkörpers 1 und schließlich in den Markraum 1' des unteren Wirbelkörpers.

Wie die Figuren zeigen, ist der Nagel 4 an seinem distalen Ende durch einen schrägen Zylinderkegelschnitt so angespitzt, dass die Spitze 4a des Nagels 4 auf der konvexen Seite der bogenförmigen Krümmung liegt (vgl. Fig. 2). Die distale Schrägfläche 4b des Nagels 4 erzeugt deshalb beim Eintreiben des Nagels in die Wirbel eine Reaktionskraft, welche die Krümmung des Nagels beim Eintreiben verstärkt derart, dass der Nagel 4 in seiner finalen Position in dem von ihm selbst erzeugten Kanal im Knochenmaterial verspannt ist. Das Eintreiben des Nagels in das Knochenmaterial verlangt also nicht notwendig den Einsatz eines gekrümmten Bohrers oder dergleichen. Die Verspannung des selbstbohrenden Nagels im Knochenmaterial bewirkt aufgrund der hohen Elastizität des Nagels eine stabile Verankerung aller beteiligten Knochenund Metallkomponenten. Der Nagel 4 durchsetzt die Endplatten 6, 8 der fusionierten Wirbelkörper 1 im Wesentlichen senkrecht zu deren Hauptebene.

Das Einführrohr 3 kann an seinem distalen Ende ebenfalls in der Art eines Zylinderkegelschnittes schräg spitz angeschliffen sein und am proximalen Ende hat das Einführrohr eine entsprechende Markierung, sodass der Arzt die Drehposition des Einführrohres beim Einschlagen in den Wirbelkörper 1 kennt. Der schräge Anschliff des Einführrohres weist in den Figuren 1 und 2 in die gewünschte Richtung des Vortriebs, hier also nach unten. Dabei wird die vordere Spitze des schrägen Anschliffs des Nagels in der Regel so gedreht, dass sie in den Figuren 1 und 2 oben liegt, um ein Austreten des Nagels aus dem Einführrohr 3 in der gewünschten Richtung (gemäß Fig. 2) zu fördern.

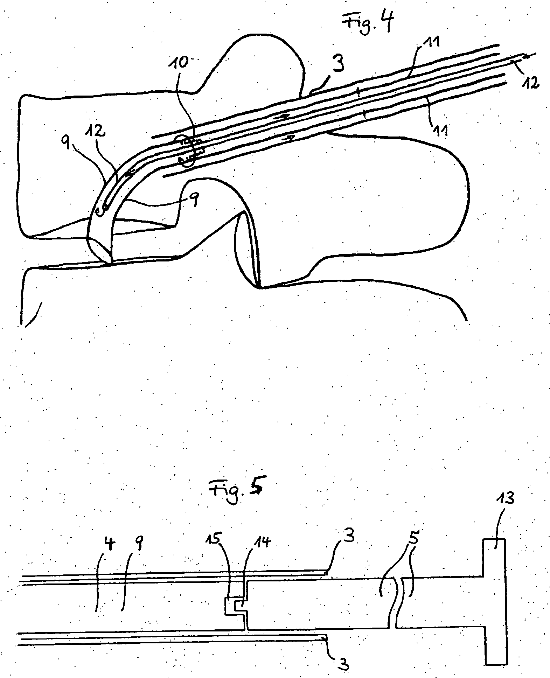

Die richtige Positionierung der Krümmung des Nagels wird weiter unten anhand der Fig. 5 näher beschrieben.

In Abwandlung des vorstehend anhand der Figuren 1, 2 und 3 beschriebenen Beispieles kann anstelle des offenen schrägen Schnittes am distalen Ende des Einführrohres ein seitliches Fenster in der Nähe des distalen Endes des Einführrohres vorgesehen sein, aus dem der Nagel austritt und in den Knochen eindringt. In diesem Fall kann das Einführrohr von seiner distalen Spitze bis zum Seitenfenster verschlossen sein.

Im Unterschied zur Vertebroplastie wird hier nach Durchdringen der Wand des Wirbelkörpers 1 das Einführrohr 3 nur wenige Millimeter weit in den Wirbelkörper eingebracht, damit der Nagel nicht die gegenüberliegende Wand des Wirbels durchdringt.

Der Krümmungsradius der oben erläuterten bogenförmigen Krümmung des Nagels hängt von den Gegebenheiten ab und kann zum Beispiel im Bereich von etwa 1 cm bis etwa 8 cm liegen.

Zur Erhöhung der Stabilität der Wirbelkörperfusion kann analog dem Einbringen eines Nagels gemäß den Figuren 1 bis 3 ein zweiter Nagel (nicht gezeigt) auf der anderen Seite des Wirbelkanals 1d (vgl. Fig. 3) transpedikulär durch beide zu verbindenden Wirbelkörper 1 eingebracht werden.

Das Vortreiben eines Nagels erfolgt unter Sichtkontrolle mittels eines bildgebenden Verfahrens. Besonders ist hierfür die Röntgen-Compurtertomographie geeignet, mit der der Zugangsweg geplant und kontrolliert werden kann. Die gewünschte Schnittebene kann errechnet werden, wodurch die räumliche Darstellung verbessert wird.

Es ist auch möglich, mit dem anhand der Figuren 1 bis 3 beschriebenen Verfahren auf einer Seite (in Fig. 3 zum Beispiel oben) zwei Nägel analog zu setzen. Hierzu wird nach vollständiger Positionierung eines ersten Nagels (wie anhand der Figuren 1 bis 3 erläutert), das Einführrohr 3 in gerader Richtung weitergetrieben in eine weiter vorne (in den Figuren 1 bis 3 links) gelegene Endposition und dort wird dann ein zweiter Nagel analog dem ersten Nagel gesetzt.

Weiterhin kann das anhand der Figuren 1 bis 3 beschriebene Verfahren dahingehend ergänzt werden, dass zusätzlich vom in den Figuren unten gelegenen Wirbel aus analog ein oder mehr Nägel von unten nach oben (bezogen auf die Figuren 1, 2) gesetzt werden.

Das Beispiel gemäß den Figuren 1 und 2 kann dahingehend abgewandelt werden, dass der Nagel proximalseitig des bogenförmig gekrümmten Abschnittes einen geraden Abschnitt aufweist, der dann beim Eintreiben in den zuvor erzeugten gebogenen Knochenkanal eine Verklemmung bewirkt, wodurch die Festigkeit der Fusion erhöht wird und eine spätere Migration des Nagels vermieden wird.

Die elastischen Biegekräfte des Nagels können dadurch beträchtlich erhöht werden, dass der Nagel aus mehreren lamellenförmigen oder konzentrisch rohrförmigen Schichten besteht, die sich bei Biegung bzw. Streckung gegeneinander verschieben können. Dies ist insbesondere bei Nägeln mit größeren Durchmessern und aus Stahl von Vorteil.

Figur 4 zeigt ein weiteres Beispiel, bei dem der Nagel 9 als Hohlnagel also mit einem Lumen ausgeformt ist. Der Nagel 9 ist an seinem distalen Ende schräg angespitzt und hinsichtlich der Krümmung und des elastischen Verhaltens sowie des Überganges von der ersten in die zweite Gestalt gilt analog das oben zum Nagel gemäß den Figuren 1 bis 3 Gesagte.

An seinem proximalen Ende ist der Nagel 9 mit einem Gewinde 10 versehen und der Pusher 11 hat an seinem distalen Ende ein entsprechendes Gewinde. Der Pusher 11 ist ebenfalls hohl und durch das Lumen des Pushers und des Nagels ist ein Schlauch 12 geschoben. Über den Schlauch 12 können Substanzen eingebracht werden, die aus dem offenen distalen Ende des Nagels 9 austreten, zum Beispiel eine kühlende Spülflüssigkeit, durch welche die Elastizitätseigenschaften des eingeschlagenen Nagels 9 verändert werden können bei Verwendung eines Materials für den Nagel, welches von der Temperatur abhängige Elastizitätseigenschaften hat, wie zum Beispiel eine Legierung mit Formgedächtnis (zum Beispiel Nitinol). Wird die Temperatur der Spülflüssigkeit so eingestellt, dass das Material des Nagels seine Elastizität verliert, kann der Nagel bei zum Beispiel einer Fehlplatzierung zurückgezogen und entfernt werden.

Ist der Nagel 9 erfolgreich implantiert, kann die Gewindeverbindung 10 gelöst und der Pusher 11 herausgezogen werden.

In Abwandlung des vorstehend beschriebenen Beispieles kann der hohle Nagel 9 an seiner distalen Spitze verschlossen sein, was das Eintreiben des Hohlnagels in das Knochenmaterial erleichtert. Kühlende Flüssigkeit tritt dann nicht in das Gewebe ein, sondern verbleibt im Nagel.

Figur 5 zeigt das proximale Ende eines Einführrohres 3 und eines Nagels 4 gemäß den Beispielen nach den Figuren 1 bis 4. Wie oben erläutert, haben die Komponenten des Systems, wie insbesondere der Nagel und der Pusher, sowie gegebenenfalls das Einführrohr Mittel, die dem benutzenden Arzt zu jedem Zeitpunkt des Eintreibens des Nagels in das Knochenmaterial die Drehposition der Komponenten anzeigen. Diese Mittel sind insbesondere jeweils Markierungen an den proximalen Enden der genannten Komponenten. Beim Beispiel gemäß Fig. 5 hat der Pusher 5 (beim Beispiel gemäß Fig. 4 der Pusher 11) einen Griff 13 und am distalen Ende einen Vorsprung 14, der in eine Ausnehmung 15 am proximalen Ende des Nagels eingreift. Solange noch diese Verbindung im Einführrohr 3 gelegen ist, lässt sich der Nagel z. B. bei einer Fehlplazierung zurückziehen. Griff 13 und Vorsprung sowie Ausnehmung sind nicht rotationssymmetrisch (also zum Beispiel stangen- bzw. schlitzförmig), sodass die Drehposition des Griffes 13 dem Benutzer Auskunft gibt über die Drehposition des Nagels. Der Benutzer kann so auch den Nagel in die gewünschte Drehposition bringen, sodass die oben beschriebene Krümmung beim Austritt aus dem Einführrohr in die gewünschte Richtung erfolgt. Auch bei diesem Beispiel (wie auch bei den anderen hier beschriebenen Beispielen) können Nagel und Pusher mit einem Lumen ausgeformt sein, um insbesondere Flüssigkeit und dergleichen bis in den distalen Bereich des Nagels einzubringen.

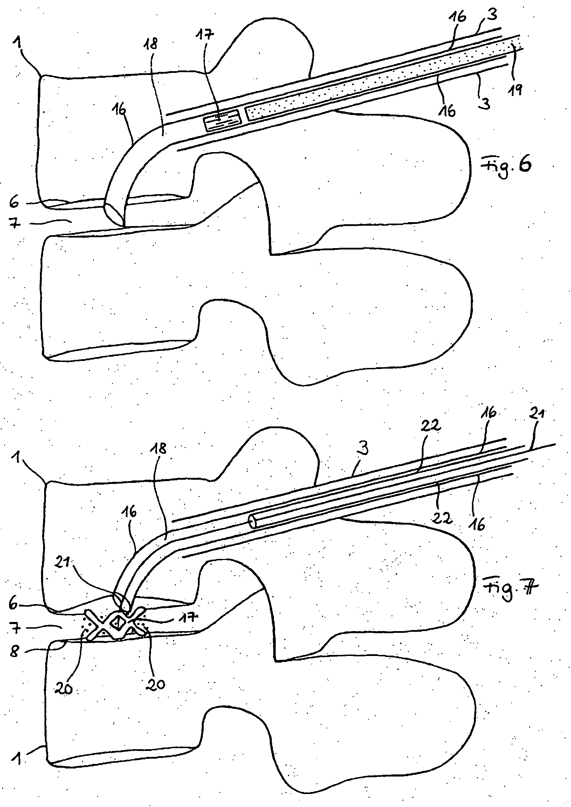

Figur 6 zeigt ein gerades Einführrohr 3 und einen hohlen Nagel 16 mit einem durchgehenden Lumen 18. Der Nagel 16 entspricht also weitgehend dem Nagel 9 des Beispieles nach Fig. 4. Beim Beispiel gemäß Fig. 6 hat der Nagel 16 aber einen geraden proximalen Abschnitt. Ein selbst-expandierender Spacer (Abstandshalter) 17 ist in das Lumen des Nagels 16 eingeführt und wird durch einen Pusher 19 in distaler Richtung vorgeschoben. Der Nagel 16 wird, wie oben beschrieben, durch leichte Hammerschläge von außerhalb des Patienten (wie bei den anderen Beispielen) in distaler Richtung vorgetrieben und nimmt die oben beschriebene erste Gestalt und die zweite Gestalt an. Der Nagel 16 wird durch den oberen Wirbelkörper 1 und dessen Grundplatte 6 in den Zwischenwirbelraum 7 eingetrieben. Mittels des Pushers 19 wird im Nagel 16 der Spacer 17 distalwärts zur Spitze des Nagels vorgeschoben. Der Pusher hat hinreichende elastische Eigenschaften, um der Krümmung des Nagels zu folgen, auch könnte der Pusher als Helix ausgeführt sein. Der Spacer 17 wird so in den Zwischenwirbelraum 7 geschoben und kann dort nach Austritt aus dem Lumen des Nagels 16 expandieren. Die Bandscheibe oder Teile derselben kann zuvor operativ entfernt worden sein.

Figur 7 zeigt eine Anordnung ähnlich Fig. 6, wobei jedoch der Pusher 22 beim Beispiel nach Fig. 7 ein Lumen aufweist, durch welches ein Führungsdraht 21 geführt ist. Der Führungsdraht 21 dient der Positionierung des Spacers 17. Fig. 7 zeigt den Zustand der Anordnung mit aus dem Nagel 16 freigesetztem Spacer 17. Im Bereich des Spacers 17 findet sich Zellmaterial 20, das sich im Lumen des Nagels 16 bei dessen Vortreiben gesammelt hatte. Dies dient zur beschleunigten Knochenneubildung im Bandscheibenfach und somit zur Wirbelfusion. Der Spacer 17 ist radial expandiert und stützt beidseitig die benachbarten Wirbelkörper 1 ab. Die axialen Enden des Spacers 17, die mit der Grundplatte 6 des oberen Wirbelkörpers bzw. der Deckplatte 8 des unteren Wirbelkörpers in Kontakt stehen, sind abgeflacht oder abgerundet, um einen flächigen Kontakt zwischen den Metallteilen und den Grund- bzw. Deckplatten zu ermöglichen.

Auch beim Beispiel nach Fig. 7 kann der Nagel 16 aus einer Legierung mit temperaturabhängigem Formgedächtnis bestehen, sodass durch Spülen mit einer geeignet temperierten Flüssigkeit die elastischen Eigenschaften des Nagels 16 so änderbar sind, dass der Nagel 16 nach Platzierung des Spacers ohne Widerstand aus dem Knochenkanal herausgezogen werden kann.

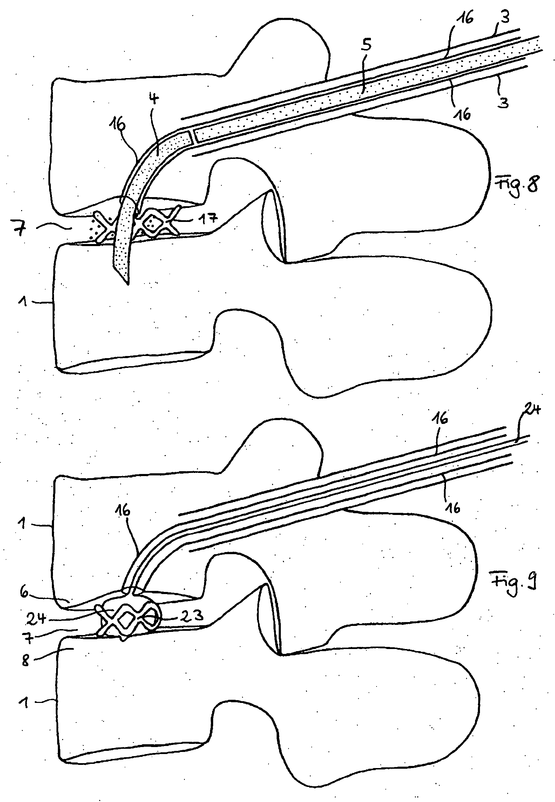

Figur 8 zeigt eine Weiterbildung der Anordnung, die von den Beispielen gemäß den Figuren 6 und 7 ausgeht. Ein Spacer 17 ist, wie vorstehend beschrieben, im Zwischenwirbelraum positioniert. Der Nagel 16 entspricht dem Nagel 9 gemäß Fig. 4, hat also die oben beschriebenen Krümmungseigenschaften und lässt sich so mit seinem schräg angeschliffen spitzen distalen Ende bis zum Beispiel in den Zwischenwirbelraum 7 einschlagen. Zusätzlich zur Ausstattung mit dem Spacer 17 sollen die beiden benachbarten Wirbelkörper fusioniert werden. Hierzu dient ein Nagel 4, der insoweit dem oben anhand von Fig. 2 beschriebenen Nagel entspricht, sodass auf die obige Beschreibung verwiesen werden kann.

Figur 9 zeigt ein Beispiel, das weitgehend den oben beschriebenen Beispielen mit Spacern entspricht, wobei der Spacer 23 gemäß Fig. 9 mittels eines Ballons 24 expandierbar ist. Die Ballonexpansion ist als solche in anderen Anwendungen bekannt. Der in der Kontur zylinderförmige Spacer besteht aus einem streckbaren Metall mit Längsschlitzen, die sich bei Expansion des Ballons verformen und rhombische Form annehmen. Die Abmessungen der Komponenten sind so, dass sich bei diesem Beispiel der Ballon 24 nur im Zwischenwirbelraum 7 ausdehnt. Es versteht sich, dass Ballon 24 und Spacer 23 beim Durchführen durch das Lumen des Nagels 16 einen komprimierten Zustand einnehmen.

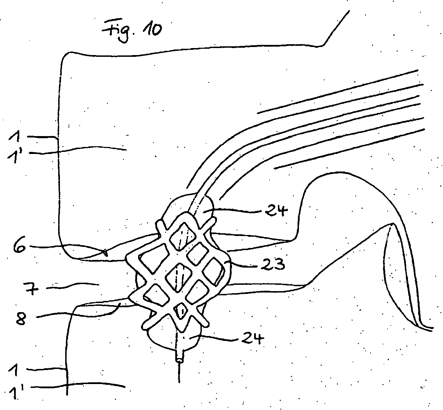

Figur 10 zeigt eine Variante der Anordnung gemäß Fig. 9, bei der der Spacer 23 aus dem Zwischenwirbelraum 7 in den Markraum 1' der benachbarten Wirbelkörper 1 hineinragt. Der Spacer 23 ist bei diesem Beispiel (in Abwandlung des Beispiels gemäß Fig. 9) axial länger als der Zwischenwirbelraum 7 und ragt deshalb über die Deckplatte 8 des unteren Wirbelkörpers 1 in dessen Markraum 1' und analog durch die Grundplatte 6 des oberen Wirbelkörpers in dessen Markraum. Der Spacer ist auf einem Ballon 24 montiert und der Ballon 24 ist wiederum axial länger als der Spacer. Beim Expandieren des Ballons 24 durch Druckbeaufschlagung dehnt sich der Spacer 23 zunächst im Zwischenwirbelraum 7 aus. Im Bereich der Deckplatte und Grundplatte kann sich der Spacer 23 aufgrund der Härte der knöchernen Strukturen nicht so stark ausdehnen, jedoch erfolgt eine weitere Ausdehnung der Enden des Spacers 23 im Markraum 1' der Wirbelkörper, da hier aufgrund der weicheren knöchernen Strukturen der Widerstand beim Expandieren geringer ist. Hierdurch wird die Abstützung und Stabilisierung der benachbarten Wirbelkörper mittels des Spacers gefördert, insbesondere wird so die Gefahr des Wirbelgleitens vermindert. Auch wird der Spacer selbst in seiner Lage stabilisiert.

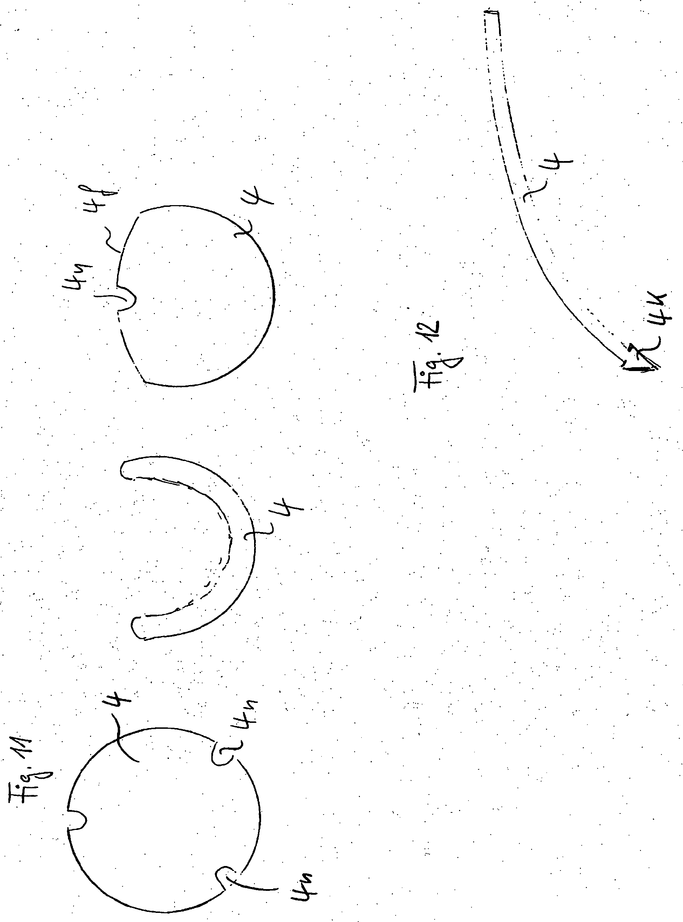

Figur 11 zeigt drei Beispiele für Nagelquerschnitte. Beim in Fig. 11 links gezeigten ersten Beispiel hat der Nagel 4 sich in Längsrichtung erstreckende Nuten 4n. Diese Längsnuten verringern den Querschnitt und Oberfläche des Nagels und somit den Widerstand beim Eintreiben des Nagels in Knochenmaterial. Auch können die Längsnuten die Biegeeigenschaften des Nagels gezielt beeinflussen.

Das in Fig. 11 mittig gezeigte Beispiel zeigt einen Nagel 4 mit U-förmigem Querschnitt. Auch mit einem solchen Querschnitt können die Biegeeigenschaften des Nagels gezielt beeinflusst werden und insgesamt hat der Nagel beim Eintreiben in einen Knochen eine reduzierte Sprengkraft, da weniger Knochenmaterial verdrängt wird. Das in Fig. 11 rechts gezeigte Beispiel eines Querschnittes eines Nagels weist auf einer Seite des ansonsten kreisförmigen Nagelquerschnitts eine Abflachung 4f auf. Auch mit einer solchen Formgebung des Nagels wird der Querschnitt und damit die Sprengkraft beim Eintreiben des Nagels in einen Knochen reduziert und es können gezielt Spannungen des Nagels im Knochenkanal erzeugt werden, sowie ein fester Sitz des Nagels im Knochenkanal.

Figur 12 zeigt einen Nagel 4 mit einem distalen Kopfabschnitt 4K, wobei der Kopfabschnitt einen Durchmesser hat, der größer ist als der Durchmesser des übrigen Nagels. Zum Beispiel kann der distale Kopfabschnitt 4K zentrisch spitz zulaufen.

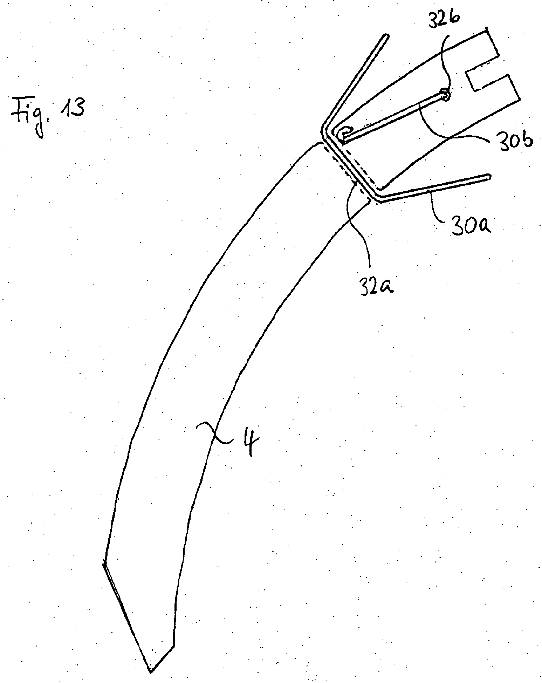

Figur 13 zeigt einen Nagel 4 mit zwei Ankern 30a, 30b. Die Anker 30a, 30b, sind etwa U-förmig und jeweils durch einen Kanal 32a bzw. 32b durch den Anker 4 geschoben, wie Fig. 13 zeigt. Beim Eintreiben des Nagels 4 durch ein Einführrohr legen sich die Anker elastisch direkt an die Außenwände des Nagels 4. Verlässt dann der Nagel 4 das Einführrohr, spreizen sich die Schenkel der Anker 30a, 30b auf und bewirken eine Verankerung des Nagels im umgebenden Knochenmaterial.

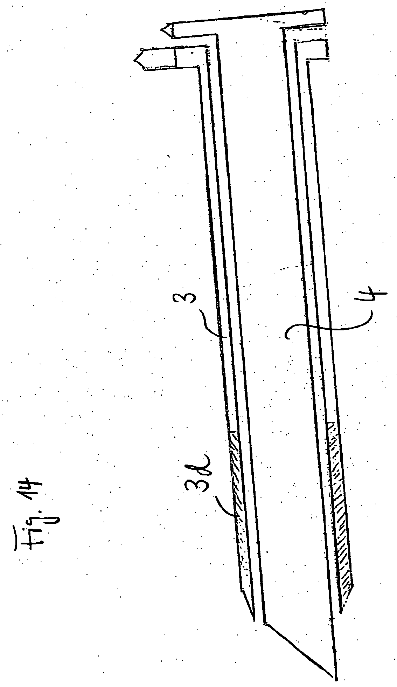

Figur 14 zeigt ein Beispiel mit einem Einführrohr 3 und einem Nagel 4. Das Einführrohr 3 hat an seinem distalen Ende einen Abschnitt 3d, der analog dem oben beschriebenen Nagel eine erste und eine zweite Gestalt annehmen kann. Beim Eintreiben in das Knochenmaterial ist der zentrale Nagel 4 wie ein Mandrin starr, sodass Einführrohr 3 mit Nagel 4 geradlinig in zum Beispiel einen Wirbelkörper oder einen Zwischenwirbelraum einbringbar sind. In der Endstellung kann der zentrale Nagel zurückgezogen werden und der Abschnitt 3d des Einführrohres 3 kann dann in die zweite Gestalt übergehen, wie oben anhand der Nägel beschrieben ist. Bei diesem Beispiel hat also das Einführrohr selbst die Funktion eines Nagels im Sinne der Patentansprüche und der Nagel 4 ist eine zusätzliche Komponente.

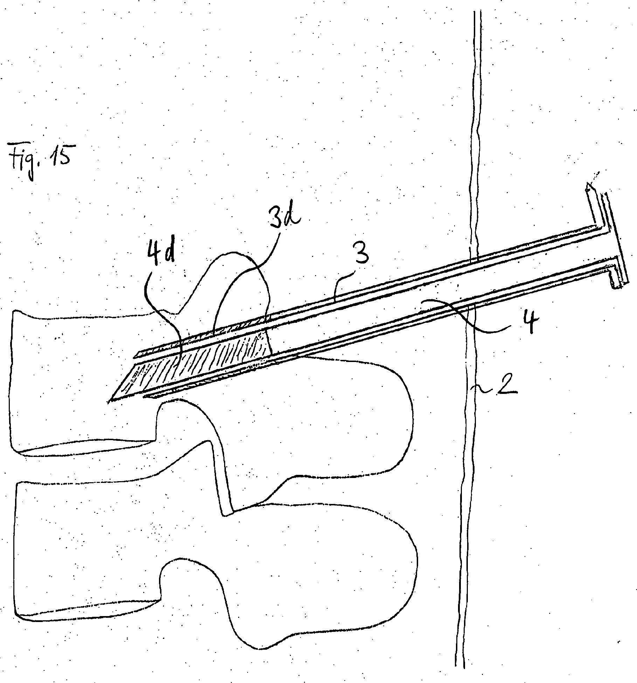

Figur 15 zeigt ein System aus Einführrohr und Nagel, wobei das Einführrohr 3 dem Einführrohr 3 gemäß Fig. 14 entspricht, also mit einem distalen Abschnitt 3d, der die oben definierte erste und die zweite Gestalt annehmen kann. Auch der Nagel 4 hat am distalen Ende einen Abschnitt 4d, der ebenfalls die oben definierte erste Gestalt und die zweite Gestalt annehmen kann. Fig. 15 zeigt auch noch einmal zur Verdeutlichung die Haut 2 und damit den perkutanen minimal-invasiven Eingriff.

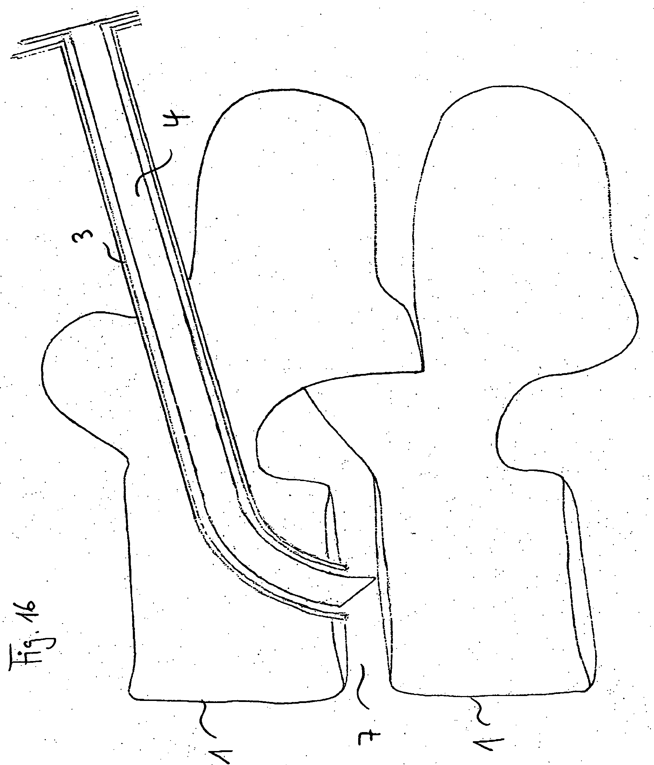

Figur 16 zeigt beispielhaft eine Endlage von Nagel und Einführrohr gemäß Fig. 15 nach dem Eintreiben. Beispielsweise kann aus dieser Stellung der Nagel 4 zurückgezogen werden, sodass das Einführrohr 3 einen Zugang zum Zwischenwirbelraum 7 eröffnet.

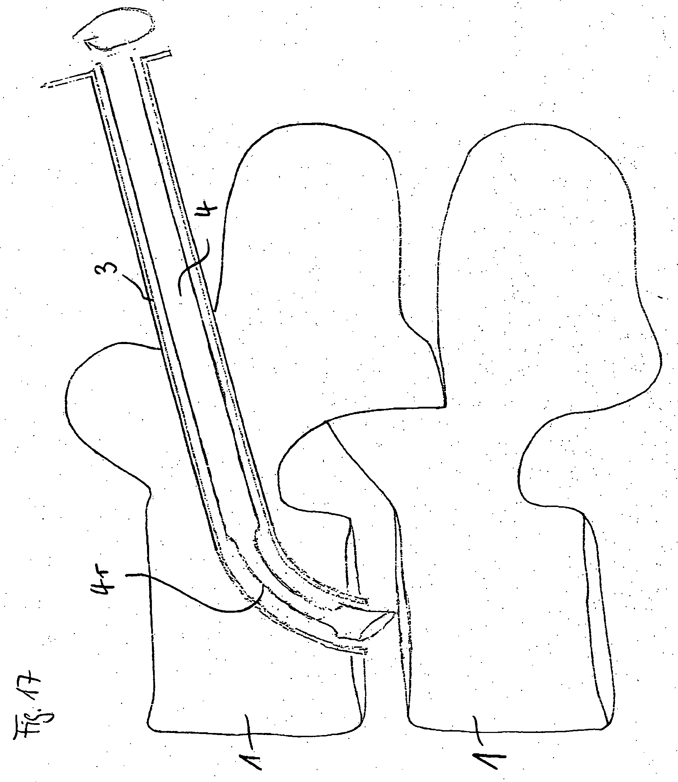

Figur 17 zeigt eine Abwandlung eines Nagels 4, der in einem Abschnitt 4r nahe seinem distalen Ende einen reduzierten Durchmesser hat. Dieser reduzierte Durchmesser liegt, wie Fig. 17 zeigt, im Bereich des Krümmungsbogens. Hierdurch wird die Handhabbarkeit des Nagels gefördert, der Nagel lässt sich leichter zurückziehen. Auch lässt sich der Nagel 4 gemäß dem Drehpfeil in Fig. 17 drehen.

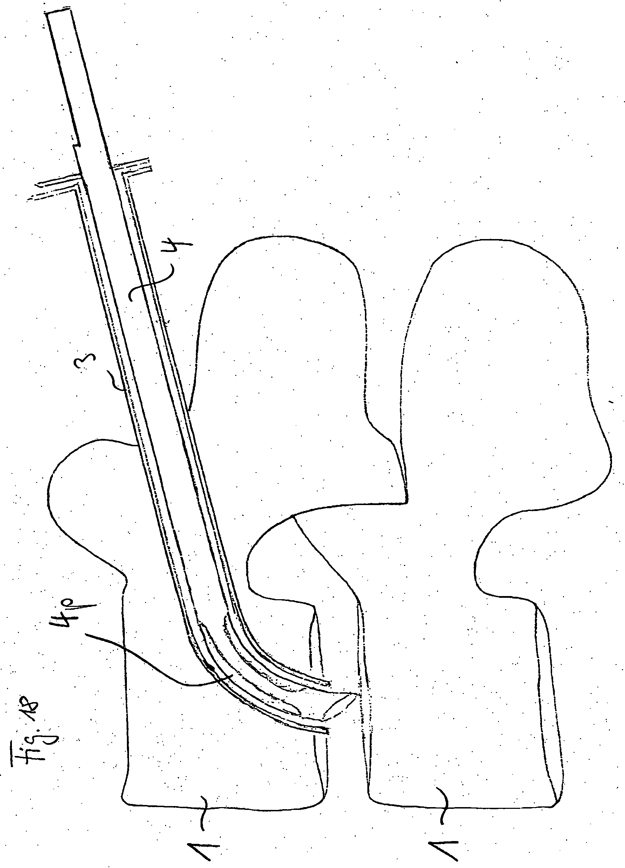

Figur 18 zeigt eine Abwandlung des Nagels gemäß Fig. 17, wobei in dem Bereich reduzierten Durchmessers nahe dem distalen Ende im Krümmungsbereich der Kern des Nagels von einem Kunststoffmantel 4p umhüllt ist. Dies fördert ebenfalls die Beweglichkeit und Handhabbarkeit des Nagels während des Einschlagens.

Alle oben und nachfolgend beschriebenen Nägel können zumindest im Wesentlichen aus einer superelastischen Metalllegierung bestehen, insbesondere Nitinol.

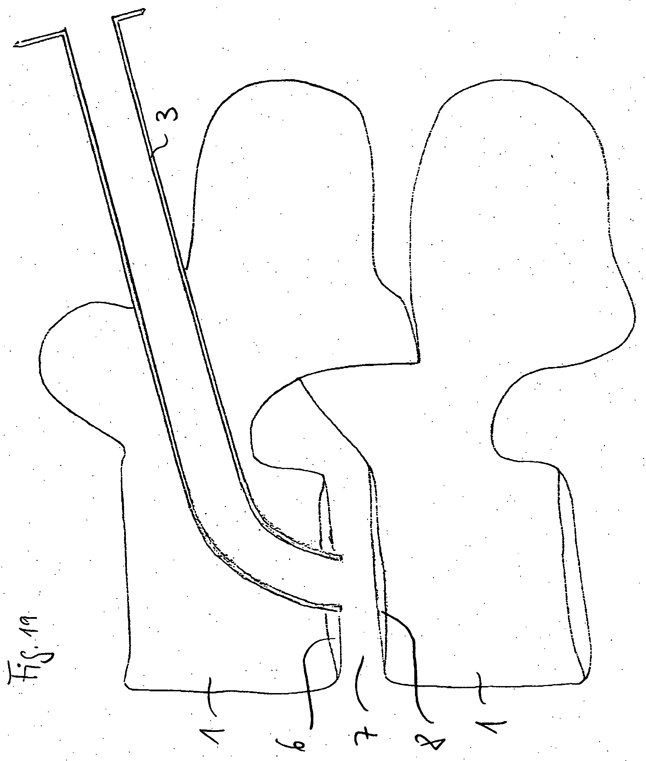

Figur 19 zeigt ein gekrümmtes Einführrohr 3 (zum Beispiel gemäß Fig. 14) im Endzustand, wobei der zuvor als Mandrin dienende Nagel entfernt ist. In dieser Endstellung bietet das Einführrohr 3 einen Zugang in den Zwischenwirbelraum 7. Bei diesem Beispiel ist also das Einführrohr ein Nagel im Sinne des Patentanspruches. Damit ist zum Beispiel eine Operation an der Bandscheibe möglich oder auch ein Absaugen. Auch möglich sind eine Gewebenentnahme aus der Bandscheibe oder Injektionen in die Bandscheibe. Auch können über das Einführrohr 3 Implantate oder ein Bandscheibenersatz eingebracht werden. Ebenfalls kann eine Drainage gelegt werden, insbesondere beim Spondylodiscitis.

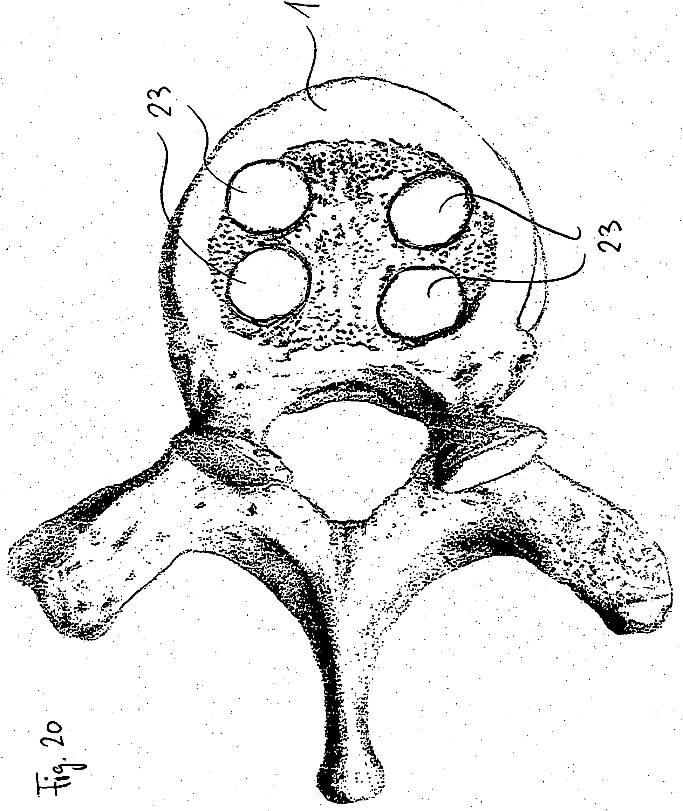

Figur 20 zeigt eine Draufsicht auf einen Wirbel, in axialer Richtung. Es sind vier Spreizkörper 23 (vgl. Fig. 10) oder vier Spreizkörper 17 (vgl. Fig. 8) positioniert.

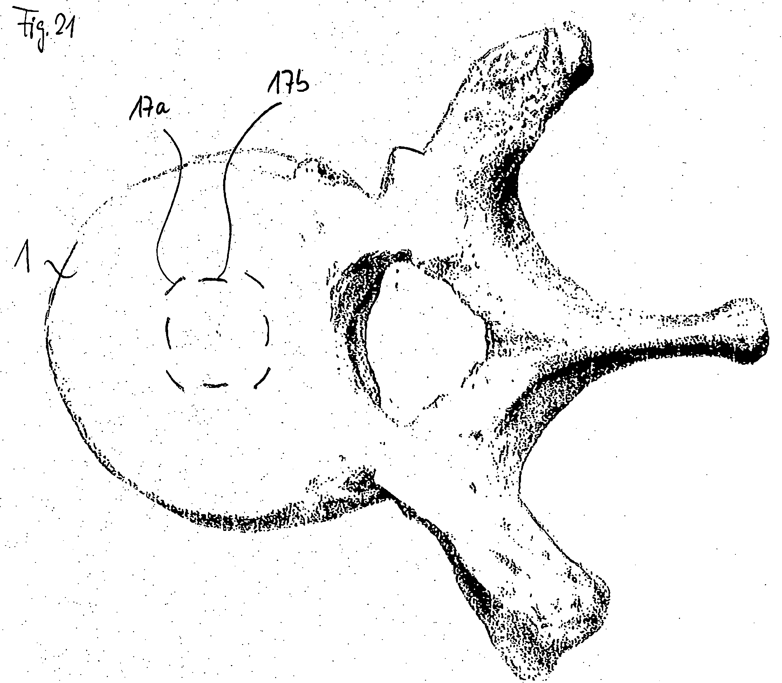

Figur 21 zeigt ebenfalls eine Draufsicht auf einen Wirbel, wobei zwischen zwei Wirbeln konzentrisch zwei Spreizkörper 17a, 17b angeordnet sind. Die Spreizkörper können den oben anhand der Fig. 8 Beschriebenen entsprechen. Auch können die Spreizkörper 23 gemäß Fig. 9 in doppelter konzentrischer Art gemäß Fig. 21 eingesetzt werden. Mit einer derartigen konzentrischen Anordnung von mehr als einem Spreizkörper kann die Spreizkraft in Bezug auf die beiden benachbarten Wirbelkörper stark erhöht werden und die Stabilität der Abstützung wird weiter gefördert.

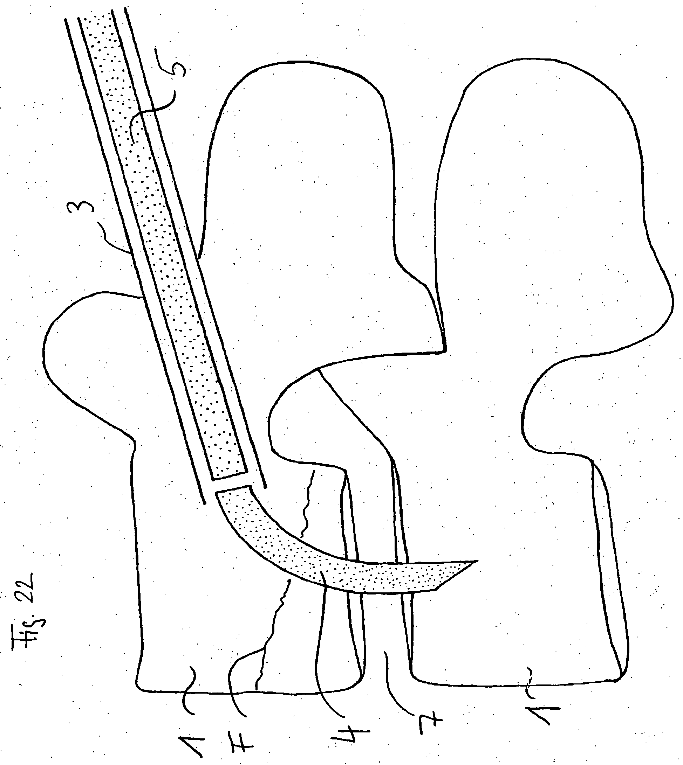

Figur 22 zeigt den Einsatz eines Nagels 4 gemäß einem der oben beschriebenen Beispiele zur Behandlung einer Fraktur F in einem Wirbelkörper 1. Der Nagel 4 wird in der beschriebenen Weise durch den Wirbelkörper getrieben und überbrückt stabilisierend die Fraktur. Dabei kann optional der Nagel 4 zugleich so weit durch den Wirbelzwischenraum 7 getrieben werden, dass er in den benachbarten Wirbelkörper 1 eindringt und so den verletzten Wirbelkörper stabilisiert. Der Nagel kann dabei vorteilhaft auch nur in die stabile Grundplatte reichen, hier liegt er fester als im Markraum.

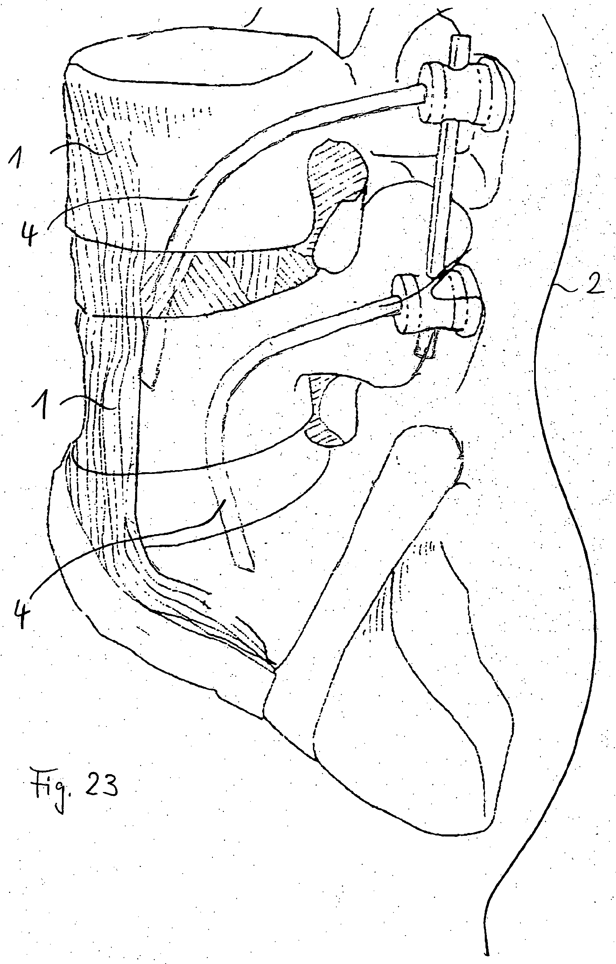

Figur 23 zeigt einen Einsatz der oben beschriebenen Nägel, bei dem mehrere Wirbelkörper 1 in der oben beschriebenen Weise von Nägeln durchsetzt sind. Dabei weisen die Nägel jeweils gerade Abschnitte auf, die so bemessen sind, dass nach der finalen Positionierung des Nagels diese etwa 10 bis 20 mm dorsal aus dem Wirbelbogen in dorsal gelegene Gewebe ragen. Dies ermöglicht eine weitere (zusätzliche) Fixation der Wirbel, wie sie aus der konventionellen orthopädischen Knochenchirurgie als solche bekannt ist. Hierzu werden die herausragenden Enden der eingebrachten Nägel dorsal mittels Muffen, Schrauben und Stahldrähten miteinander verbunden. Hierdurch entsteht eine weitere Fusionskomponente mit Abstand zur ersten Fusionskomponente (Nägel), was insgesamt die Stabilität der Fusion deutlich erhöht.

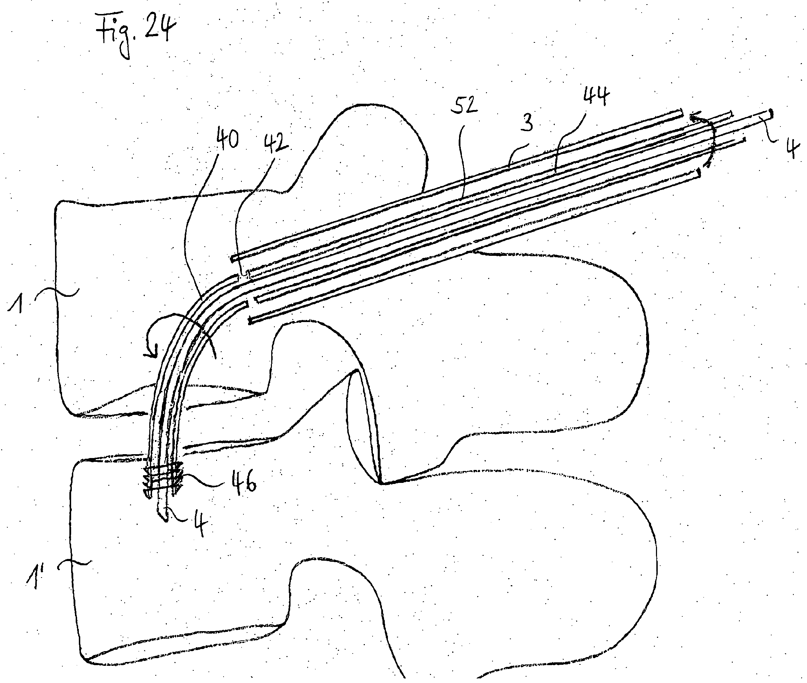

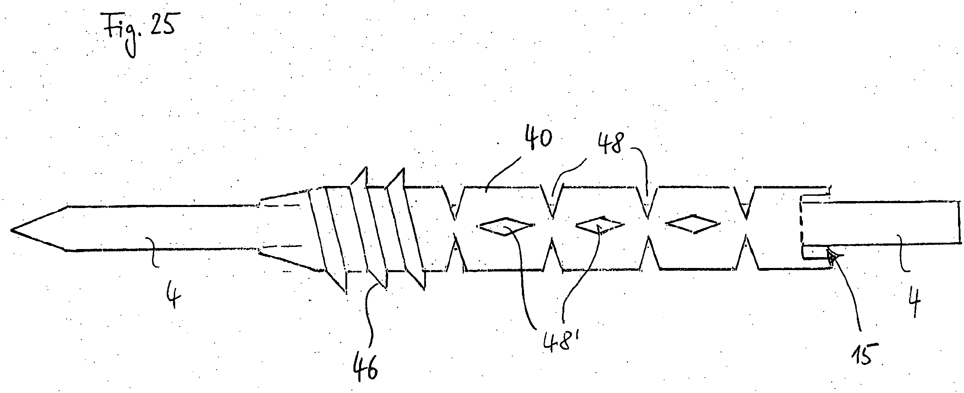

Die Figuren 24 und 25 zeigen ein erfindungsgemäßes Ausführungsbeispiel eines Nagels zum perkutanen Einbringen in z. B. Wirbelkörper. Bei diesem Ausführungsbeispiel ist wieder ein Einführrohr 3 vorgesehen. Ein innerer Nagel 4 dient hier als Führung für einen äußeren Nagel 40. Der äußere Nagel 40 ist flexibel, er ist relativ leicht biegbar. Der Hohlnagel kann durch eine einzelne helixartige Struktur in seinem Mantel durchzogen sein, sodass bei Drehung der Durchmesser des Hohlnagels kleiner wird. Hierdurch lässt er sich leichter einschrauben, da die Reibung mit dem umgebenden Gewebe geringer wird. Danach dehnt sich der Nagel im Durchmesser wieder auf und dies garantiert einen festen Sitz. Die Flexibilität wird zum Beispiel mit Einkerbungen 48 oder Schlitzen in der Wandung des hohlen äußeren Nagels 40 erreicht.

Gemäß Figur 24 wird der hohle äußere Nagel 40, wie oben beschrieben, über das Einführrohr eingeführt. Dabei dient der innere Nagel 4 als Führung. Ein Aktuator 52 ist ebenfalls in das Einführrohr 3 eingeführt. Der Aktuator 52 ist über eine Kuppelung 42 (in Fig. 24 nicht im Einzelnen dargestellt) mit dem äußeren hohlen Nagel 40 für eine Drehbewegung und Druckbeaufschlagung gekoppelt.

An seinem distalen Ende ist der äußere hohle Nagel 40 mit einem Gewinde 46 versehen, sodass er bei Drehung in Knochengewebe einschraubbar ist. Das Gewinde kann sich auch über einen längeren Abschnitt des Nagels erstrecken, insbesondere über dessen gesamte Länge. Bei diesem System wird zunächst der mit einem Schraubengewinde versehene Hohlnagel 40 mit verbundenem Aktuator 52 bis an das distale Ende des Einführrohres geschoben, wobei der innere Nagel 4 als Führung dient. Dann wird der innere Nagel 4 bis in den unteren Wirbelkörper 1' eingeschlagen und danach wird der longitudinal flexible Hohlnagel 40 durch die knöchernen Strukturen und die Bandscheibe gemäß Fig. 24 in z. 8. den unteren Wirbelkörper 1' eingeschraubt, sodass die beiden benachbarten Wirbelkörper 1, 1' miteinander verbunden werden. Dabei ist es auch möglich, dass das Schraubgewinde den inneren Nagel (Führungsnagel) zeitweise überragt und dieser nachträglich eingeschlagen wird. Durch die Schraubung entsteht ein fester Eingriff ohne Lockerungs- oder Migrationsgefahr. Bei diesem Ausführungsbeispiel hat der Hohlnagel 40, der im oben erläuterten Sinne die erste und die zweite Gestalt annehmen kann, an seinem distalen Ende nicht notwendig eine Spitze, sondern ein Gewinde. Optional kann ein innerer Nagel (ohne Spitze) in den äußeren, am distalen Ende ein Gewinde aufweisenden Nagel eingesetzt werden, um die Verbindung zu verstärken.

Der innere Nagel kann durch einen geraden biegsamen elastischen Nagel in der Position gemäß Figur 24 ausgetauscht werden. Dieser weitere Nagel wird dann weiter in den Wirbelkörper 1' geschlagen bis dieser den nächsten Wirbelkörper (nicht gezeigt) erreicht. Dann wird der hohle Nagel 40 mit seinem Gewinde 46 über den hinzugefügten Nagel geschraubt, um so eine Verbindung von mehreren Wirbelkörpern (hier drei Wirbelkörper) herzustellen. Der hohle Nagel ist weiterhin an seinem proximalen Ende im obersten Wirbel 1 gebogen.

Figur 25 zeigt die distalen Bereiche des inneren Nagels 4 und des äußeren Nagels 40. Um die oben genannte longitudinale Flexibilität des äußeren Nagels 40 zu erreichen, ist er mit Einkerbungen 48, 48' versehen, die, wie dargestellt, um 90° in Drehrichtung gegeneinander versetzt sind. Die Abmessungen und Durchmesser der Komponenten sind so gewählt, dass der an seinem Ende mit dem Gewinde 46 versehene äußere Nagel 40 auf dem feststehenden inneren Nagel 4 mittels der Kupplung 15 drehbar ist.

## Patentansprüche

1. Medizinische Vorrichtung, aufweisend
- einen äußeren Nagel (40), der ausgebildet ist zum perkutanen Einbringen in zumindest einen Knochen,
- ein Einführrohr (3), in dem der äußere Nagel (40) positionierbar ist derart, dass er in dem Einführrohr eine erste Gestalt annimmt, in der er gerade ist, und eine zweite Gestalt, in der er außerhalb des Einführrohres eine gekrümmte Form annimmt, wobei das Einführrohr (3) gerade ist,
**dadurch gekennzeichnet, dass**
- der äußere Nagel (40) hohl ist,
- der äußere Nagel an seinem distalen Ende oder über seine gesamte Länge ein Gewinde (46) hat, und dass
- ein innerer Nagel (4) als Führung für den äußeren Nagel (40) vorgesehen ist.

2. Vorrichtung nach Anspruch 1, **dadurch gekennzeichnet, dass** der äußere Nagel aufgrund einer internen Eigenspannung von der ersten Gestalt in die zweite Gestalt übergeht.

3. Vorrichtung nach Anspruch 2, **dadurch gekennzeichnet, dass** die Eigenspannung eine elastische Biegespannung ist.

4. Vorrichtung nach einem oder mehreren der vorhergehenden Ansprüche, **dadurch gekennzeichnet, dass** die Eigenspannung temperaturabhängig ist.

5. Vorrichtung nach einem oder mehreren der vorhergehenden Ansprüche, **dadurch gekennzeichnet, dass** der äußere Nagel (40) bei Drehung im Durchmesser kleiner wird.

6. Vorrichtung nach einem oder mehreren der vorhergehenden Ansprüche, **dadurch gekennzeichnet, dass** der äußere Nagel aus einem superelastischen Metall, insbesondere Nitinol, besteht.

7. Vorrichtung nach einem oder mehreren der vorhergehenden Ansprüche, **dadurch gekennzeichnet, dass** der äußere Nagel (40) ausgebildet ist zum perkutanen Einbringen in zumindest einen Wirbelkörper.

8. Vorrichtung nach Anspruch 1 **dadurch gekennzeichnet, dass** der innere Nagel (4) eingerichtet ist, im äußeren Nagel (40) verbleibend eine Verbindung zwischen Knochen zu verstärken.

9. Vorrichtung nach einem oder mehreren der vorhergehenden Ansprüche, **dadurch gekennzeichnet, dass** der äußere Nagel (40) mit Einkerbungen (48, 48') versehen ist, die in Drehrichtung gegeneinander versetzt sind.

10. Vorrichtung nach einem oder mehreren der vorhergehenden Ansprüche, mit einem selbst-expandierenden Abstandshalter (17), der eingerichtet ist, um in einen Zwischenwirbelraum (7) geschoben zu werden.

## Claims

1. Medical device, comprising
- an outer nail (40), configured for percutaneous introduction into at least one bone,
- an introduction tube (39 in which the outer nail (40) can positioned such that, in the introduction tube, it takes a first shape in which it is straight and, outside the introduction tube, it takes a second shape in which it is bended, wherein in the introduction tube (3) it is straight,
**characterized in that**
- the outer nail (40) is hollow,
- the outer nail comprises at its distal end or along its entire length a thread (46), and
- an inner nail (4) is provided as a guide for the outer nail (40).

2. Device according to claim 1, **characterized in that** the outer nail passes from the first shape into the second shape because of an internal stress.

3. Device according to claim 2, **characterized in that** the internal stress is a elastic bending stress.

4. Device according to one or more of the preceeding claims, **characterized in that** the internal stress is dependent from temperature.

5. Device according to one or more of the preceeding claims, **characterized in that** the outer nail (40) is reduced in diameter upon rotation.

6. Device according to one or more of the preceeding claims, **characterized in that** the outer nail is made of super elastic metal, in particular nitinol.

7. Device according to one or more of the preceeding claims, **characterized in that** the outer nail (40) is configured for percutaneous introduction into at least a vertebral body.

8. Device according to claim 1, **characterized in that** the inner nail (4), when remaining in the outer nail (40), is configured to improve a connection between bones.

9. Device according to one or more of the preceeding claims, **characterized in that** the outer nail (40) comprises notches (48, 48') which are off-set in rotational direction.

10. Device according to one or more of the preceeding claims, comprising a self-expanding spacer (17) configured to be inserted into a space (7) between vertebral bodies.

## Revendications

1. Dispositif médical comprenant
- un clou extérieur (40) qui est agencé pour être inséré par voie percutanée dans au moins un os,
- un tube d'introduction (3), dans lequel le clou extérieur (40) peut être positionné de telle façon qu'il adopte, dans le tube d'introduction, une première forme dans laquelle il est droit, et une deuxième forme dans laquelle il adopte, à l'extérieur du tube d'introduction, une forme courbe, le tube d'introduction (3) étant droit,
**caractérisé en ce que**
- le clou extérieur (40) est creux,
- le clou extérieur est muni d'un filetage (46) à son extrémité distale ou sur toute sa longueur, et **en ce que**
- un clou intérieur (4) est prévu comme guide pour le clou extérieur (40).

2. Dispositif selon la revendication 1, **caractérisé en ce que** le clou extérieur passe de la première forme à la deuxième forme sous l'effet d'une contrainte interne.

3. Dispositif selon la revendication 2, **caractérisé en ce que** la contrainte interne est une contrainte de flexion élastique.

4. Dispositif selon l'une ou plusieurs des revendications précédentes, **caractérisé en ce que** la contrainte interne est fonction de la température.

5. Dispositif selon l'une ou plusieurs des revendications précédentes, **caractérisé en ce que** le clou extérieur (40) devient plus petit en diamètre lors de la rotation.

6. Dispositif selon l'une ou plusieurs des revendications précédentes, **caractérisé en ce que** le clou extérieur est constitué d'un métal superélastique, en particulier de nitinol.

7. Dispositif selon l'une ou plusieurs des revendications précédentes, **caractérisé en ce que** le clou extérieur (40) est agencé pour être inséré par voie percutanée dans au moins un corps vertébral.

8. Dispositif selon la revendication 1, **caractérisé en ce que** le clou intérieur (4) est agencé pour renforcer une liaison entre des os en restant dans le clou extérieur (40).

9. Dispositif selon l'une ou plusieurs des revendications précédentes, **caractérisé en ce que** le clou extérieur (40) est pourvu d'entailles (48, 48') qui sont décalées les unes par rapport aux autres dans le sens de rotation.

10. Dispositif selon l'une ou plusieurs des revendications précédentes, comportant un espaceur auto-expansible (17) qui est agencé pour être poussé dans un espace intervertébral (7).
